# EUROPEAN PATENT SPECIFICATION

(11) **EP 4 367 637 B1**
(45) Date of publication and mention of the grant of the patent: **04.11.2026**
(21) Application number: 23729163.8
(22) Date of filing: 26.04.2023
(51) Int. Cl.: G06T 7/80, G06T 5/50, G06T 5/80

(54) **ONLINE STEREO CALIBRATION**
ONLINE-STEREOKALIBRIERUNG
ÉTALONNAGE STÉRÉO EN LIGNE

(30) Priority: 29.04.2022 US 202217733350
(43) Date of publication of application: 15.05.2024
(73) Proprietor: Cilag GmbH International, 6300 Zug (CH)
(72) Inventor: HANSEN, Steen M., 2200 Copenhagen (DK); STOKHOLM, Mathias B., 2200 Copenhagen (DK); BRUUN, Johan M.V., 2200 Copenhagen (DK); VAN DIETEN, Job, 2200 Copenhagen (DK); KRISTENSEN, Marco D. F., Santa Clara, California 95054 (US)
(74) Representative: Carpmaels & Ransford LLP
(86) International application number: PCT/IB2023/054277
(87) International publication number: WO 2023/209581

(56) References cited:
- PRATT PHILIP ET AL: "Practical Intraoperative Stereo Camera Calibration", 14 September 2014, ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 667 - 675, ISBN: 978-3-319-10403-4, XP047521381
- L. MAIER-HEIN ET AL: "Optical techniques for 3D surface reconstruction in computer-assisted laparoscopic surgery", MEDICAL IMAGE ANALYSIS, vol. 17, no. 8, 3 May 2013 (2013-05-03), GB, pages 974 - 996, XP055249579, ISSN: 1361-8415, DOI: 10.1016/j.media.2013.04.003

## Description

### BACKGROUND

Surgical systems may incorporate an imaging system, which may allow the clinician(s) to view the surgical site and/or one or more portions thereof on one or more displays such as a monitor. The display(s) may be local and/or remote to a surgical theater. An imaging system may include a scope with a camera that views the surgical site and transmits the view to a display that is viewable by the clinician. Scopes include, but are not limited to, laparoscopes, robotic laparoscopes, arthroscopes, angioscopes, bronchoscopes, choledochoscopes, colonoscopes, cytoscopes, duodenoscopes, enteroscopes, esophagogastro-duodenoscopes (gastroscopes), endoscopes, laryngoscopes, nasopharyngo-neproscopes, sigmoidoscopes, thoracoscopes, ureteroscopes, and exoscopes.
Philip Pratt et al., "Practical Intraoperative Stereo Camera Calibration", 14 September 2014, Advances in Databases and Information Systems, Lecture Notes in Computer Science, Springer International Publishing, Cham, pp. 667 - 675, ISBN: 978-3-319-10403-4 discloses a calibration scheme comprising modeling stereo camera parameters as functions of a single 'focus position' variable, fitted to a sequence of pre-calibrated values across the desired focus range, wherein the calibration scheme provides a one-time fit over the whole focus range.
L. Maier Hein et al., "Optical techniques for 3D surface reconstruction in computer-assisted laparoscopic surgery", Medical Image Analysis, vol. 17, no. 8, 3 May 2013, pages 974 - 996, GB, ISSN: 1361-8415, DOI: 10.1016/j.media.2013.04.003 discloses intra-operative 3D reconstruction in laparoscopic surgery.

Surgical imaging systems may also involve stereo vision, which can allow for 3D reconstruction of patient anatomy captured by the imaging systems. Scene reconstruction or 3D reconstruction is a process of capturing the shape and appearance of real objects. Thus, allowing medical professionals to use a scope-based imaging system to capture, reconstruct, and track an internal area of a patient as well as any tools present in the images.

While various kinds of surgical instruments and image captures systems have been made and used, it is believed that no one prior to the inventor(s) has made or used the invention described herein.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1 depicts an illustrative flow diagram showing undistortion and rectification in a stereo image system;
FIG. 2 depicts an illustrative flowchart of a method which could be performed by a surgical visualization system based on this disclosure;
FIG. 3 illustrates error which may exist due to calibration drift, calibration error, or image localization error;
FIG. 4 depicts a pair of example corrected images such as could be derived from raw images captured by image sensors in a stereo imaging system;
FIG. 5 depicts a scenario in which scan lines indicate the presence of an offset;
FIGS. 6A-6C depict geometry of tools which may be used in recalibration in some embodiments;
FIG. 7 depicts a schematic view of a surgical visualization system; and
FIG. 8 depicts a schematic diagram of a control system.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The invention is defined in independent claims 1, 10, 11 and 14. Advantageous embodiments are defined in the dependent claims. The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

For clarity of disclosure, the terms "proximal" and "distal" are defined herein relative to a surgeon, or other operator, grasping a surgical device. The term "proximal" refers to the position of an element arranged closer to the surgeon, and the term "distal" refers to the position of an element arranged further away from the surgeon. Moreover, to the extent that spatial terms such as "top," "bottom," "upper," "lower," "vertical," "horizontal," or the like are used herein with reference to the drawings, it will be appreciated that such terms are used for exemplary description purposes only and are not intended to be limiting or absolute. In that regard, it will be understood that surgical instruments such as those disclosed herein may be used in a variety of orientations and positions not limited to those shown and described herein.

Furthermore, the terms "about," "approximately," and the like as used herein in connection with any numerical values or ranges of values are intended to encompass the exact value(s) referenced as well as a suitable tolerance that enables the referenced feature or combination of features to function for the intended purpose(s) described herein.

Similarly, the phrase "based on" should be understood as referring to a relationship in which one thing is determined at least in part by what it is specified as being "based on." This includes, but is not limited to, relationships where one thing is exclusively determined by another, which relationships may be referred to using the phrase "exclusively based on."

### I. Calibration of Stereo Images in Computer Vision

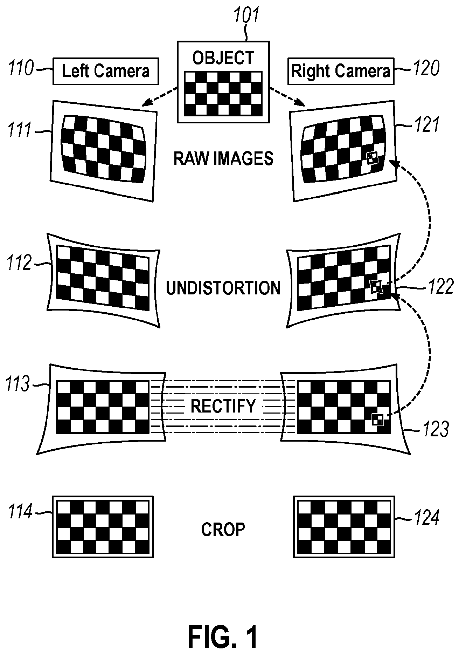

FIG. 1 depicts an illustrative flow diagram showing the process of undistortion and rectification based on parameters that are determined during calibration in a stereo image system, such as disclosed herein. In some embodiments, and as shown, a single object 101 may be captured by two different (e.g., left 110 and right 120) image capture devices (e.g., cameras). As would be understood by one of ordinary skill in the art, capturing images of an object 101 from different locations and/or angles (e.g., from a left side 110 and a right side 120) relative to that object allows for stereo vision. As used herein, stereo vision means capturing at least two images that allow for computing of depth of field based on disparity between the images. As discussed herein, computing the depth of field and/or disparity may involve matching up features in the at least two images.

It should be understood that various calibration methods and/or images may be used. Thus, although FIG. 1 only shows using an image of a checkerboard pattern, it should be understood that other calibration images may be used as well. As shown in FIG. 1, a camera calibration method using the checkerboard pattern allows for high-precision calibration under complicated illumination. In general, calibration of stereo images in computer vision is well known in the art and should be understood by one of ordinary skill. In one example embodiment, the calibration involves a few general steps. First, the system may detect checkerboard corners, for example using an improved Harris corner detector. Regardless of algorithm, the system may try to find the specified number of checkerboard corners in each input image.

Next, the system may perform the optional step of estimating undistortion coefficients. For example, in some embodiments, the algorithm will solve for coefficients that minimize standard lens distortion. Although this does not undistort the original photographs or video, these coefficients can then be used in the calibration and reconstruction process to minimize error due to lens distortion. The system may also estimate a direct linear transformation. For example, the algorithm may estimate a set of optimized direct linear transformation coefficients that can be used to reconstruct points and curves from each view into 3D. Finally, the system may estimate calibration accuracy. In some embodiments, the algorithms may estimate the calibration, using a separate set of images within the calibration set (if a sufficient number of images are supplied).

In some embodiments, and as shown, raw images (e.g., 111 and 121) may be captured from the image capture devices. Due to the nature of surgical scopes and their operating conditions, the captured raw images (111 and 121) may be deformed by radial and tangential distortion, such as may be caused by the lenses of image capture devices. Accordingly, in some embodiments, the raw images may be transformed by the system, such as, for example, by removing distortion 112 and 122.

Typically, an image processing system creates a matrix that corresponds to each pixel in an image and through interpolation, the system can then remove distortion 112 and 122. Once the distortion is removed, the images may be morphed/deformed so that the image planes become coplanar and row-aligned and thus rectify the images 113 and 123. Once images have been rectified, the system may crop them 114 and 124 in such a way as to emphasize the common area between both images. In some embodiments, the calibration may also consider the alignment between the two stereo image capture devices. As part of the process of analyzing and correcting the raw images 111 and 121 (e.g., removing the distortion, rectifying, and cropping the image) a system can collect information associated with each step for future use. Stated differently, once the system has determined how to correct one set of captured images, it can correct future images captured by the same stereo image device using the collected metrics.

### II. Online/real-time Stereo Calibration

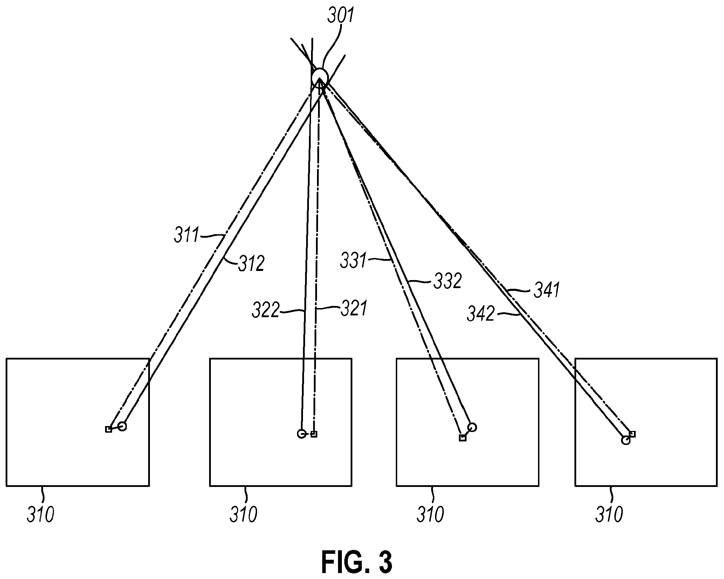

As discussed in the prior section, a system such as may be implemented based on this disclosure may obtain metrics associated with the stereo image device (e.g., initial calibration data) for use in deriving three dimensional information, such as triangulations of individual points or a three dimensional reconstruction of the scene as a whole, from subsequently captured images. However, in some cases, a system implemented based on this disclosure may not simply use initial calibration data, and instead may include features which address the possibility that a camera's characteristics may not remain constant after its initial calibration. For example, in the case of cameras used in the context of minimally invasive surgery, the accuracy of a stereo camera's calibration may be impacted by activities such as sterilization, or may drift during storage and/or transportation, or even during a procedure itself. An illustration of the types of errors this may introduce is shown in FIG. 3, as shown in that figure, a plurality of image capture devices 310, 320, 330, and 340 may be used to determine a computed 3D point 301. However, because the calibration of those devices may drift, the calibrated projection lines 311, 321, 331, 341 of point 301 may drift away from the actual point, as shown with lines 312, 322, 332 and 342. To account for this type of error, referred to as "reprojection error," some embodiments may provide for monitoring and/or updating of calibration during a procedure so that the accuracy of three dimensional reconstructions of the surgical site are not compromised by incorrect calibration.

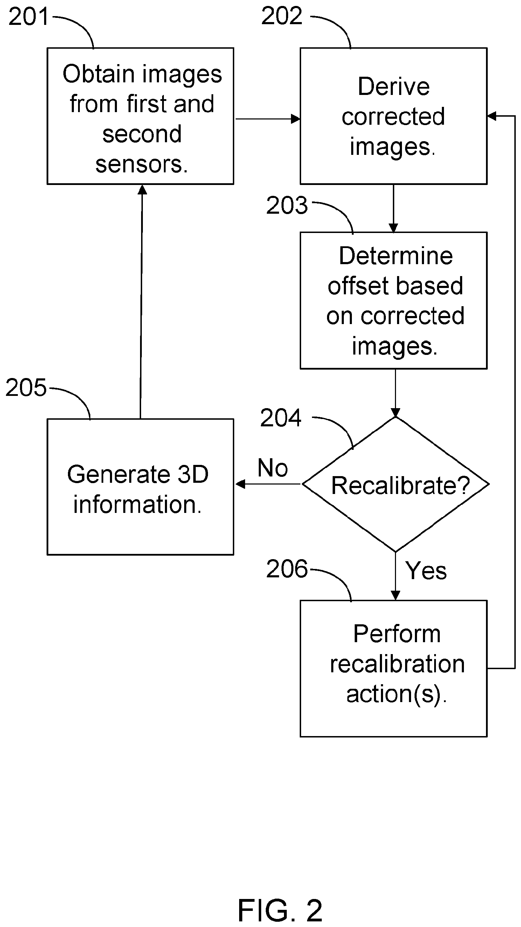

Referring now to FIG. 2, that figure depicts an illustrative flowchart of a method which could be performed by a surgical visualization system based on this disclosure. In some embodiments, a system implemented based on this disclosure may obtain 201 images from first and second image sensors (e.g., left and right sensors from a stereo camera). These images may then be used to derive 202 corrected images (e.g., rectified images) using calibration data such as could be created in a process such as described above. This calibration data may include both intrinsic and extrinsic parameters/characteristics. Extrinsic parameters are parameters that identify the transformation between a camera reference frame, a world reference frame, and the relative transformation between the two cameras. Determining these parameters may involve determining a translation vector between the relative positions of the origins of the two reference frames and also determining the rotation matrix that brings the corresponding axes of the two frames into alignment. Intrinsic parameters generally characterize the optical, geometric, and digital characteristics of the camera, such as, for example, the perspective projection (i.e., focal length), the transformation between image plane coordinates and pixel coordinates, and the geometric distortion introduced by the optics.

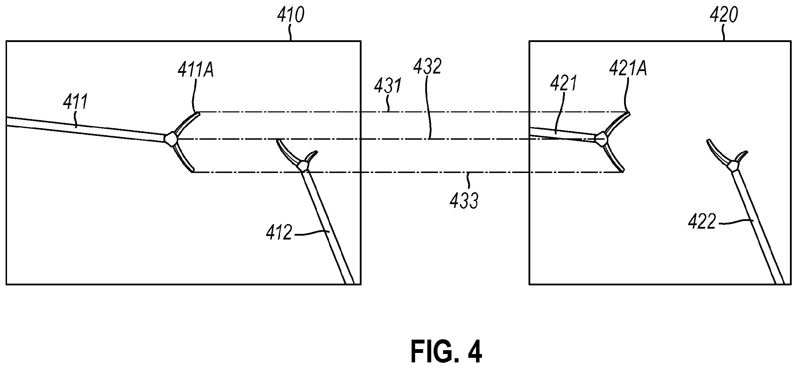

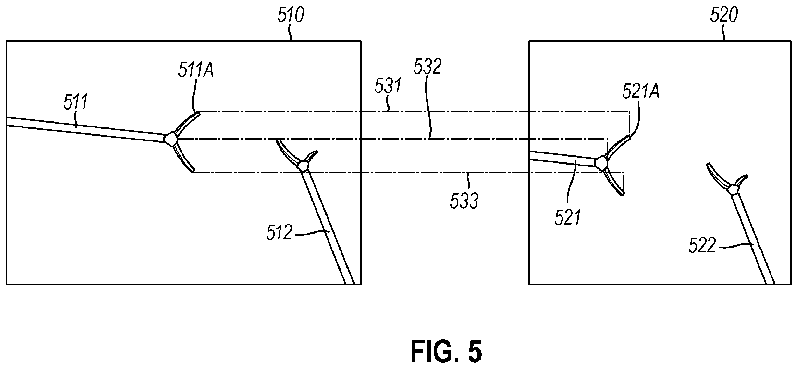

Once the corrected images had been derived 202, those images may be used to determine 203 an offset which, as shown, could be used in a subsequent determination 204 of whether it would be appropriate to recalibrate the image sensors. Various non-limiting examples of how this type of offset determination 203 may be performed are described below in the context of FIGS. 4 and 5. Although the use of scanlines is primarily discussed herein, it should be understood that alternative embodiments may exist, such as, for example, using tool geometry to calculate offsets (discussed herein), and/or using a neural network AI to predict bad calibration.

Referring now to FIG. 4, a pair of example corrected images such as could be derived from images captured by image sensors in a stereo imaging system is shown. Specifically, FIG. 4 shows a first corrected image 410 derived (e.g., though the use of calibration data as described previously in the context of FIG. 1) from an image captured by a first image sensor (e.g., a left camera) and a second corrected image 420 derived from an image captured by a second image sensor (e.g., a right camera). In some embodiments, and as shown, the first corrected image 410 may contain a view of one or more tools 411 and 412 and the second corrected image 420 may contain an alternative view of the one or more tools 421 and 422. In minimally invasive surgery, surgeons are typically using laparoscopic tools to perform internal surgery. Thus, described herein, some embodiments of the disclosed technology the system may utilize the known 3D shapes of tools to determine if calibration is needed, and in some cases, carry out the calibration. Other embodiments may also exist, for example, carrying out an online calibration check and/or recalibration based on images that are not known (e.g., tools without known geometry, patient anatomy, etc.).

In some embodiments, a portion, or all, of one or more tools (e.g., the tip of a tool 411A and 421A) may be identified by the system. For example, the system may have been given a surgical plan that includes a listing of all possible tools, and their specific characteristics, which would be used during the procedure. In an alternative example, the system may have, or obtain, a database of surgical tools, including their specific characteristics. Stated differently, the system may know, prior to analyzing the images, what tools are expected to be present in the stereo images and what their specific characteristics should be. Specifically, the system may know or obtain the tool size, shape, color, construction material, and the like. In a further embodiment, the tool may have an identifying marker that the system can use to associate it with a known tool's characteristics. In an alternative embodiment, the system may use a deep-learning neural network that has been trained on various surgical tools to track and/or identify the tools.

The tool locations, combined with the known information regarding their characteristics may then be used to identify features which can be used in determining 203 an offset. This may be done, for example, by evaluating scan lines from a feature of a tool (e.g., a distal tip) in a corrected image derived from an image captured with a first image sensor with that feature in a corresponding (e.g., derived from a simultaneously captured image) corrected image from a second image sensor. For example, if the image sensors are horizontally offset from each other, then alignment may be evaluated by determining if the corresponding features of a tool shown in corrected images derived from images captured by both first and second image sensors are vertically registered with each other, such as shown in FIG. 4. Thus, in a scenario corresponding to FIG. 4, a system implemented based on this disclosure may conclude that no calibration drift has occurred, or that the amount of calibration drift is negligible. However, in some cases, such as shown in FIG. 5, the scan lines 531, 532, and 533 may indicate the presence of an offset (e.g., a vertical offset as shown) between the first image 510 and the second image 520, indicating that at least some level of calibration drift has occurred. In some embodiments, other features/characteristics may also be compared. For example, a pivot point of a tool, a tip other than a distal tip, etc. may be used in determining an offset. As shown in FIG. 4, one or more scan lines (e.g., 431, 432, and 433) may be used to align the identified tool features/characteristics (e.g., 411A and 421A) of tool 411 and/or tool 412 in the first image 410 and of tool 421 and/or tool 422 in the second image 420.

Returning now to the discussion of FIG. 2, after an offset has been determined 203, a further determination 204 may be made as to whether it would be appropriate to recalibrate the stereo camera used to capture images. This may be done, for example, by determining if an offset indicated that there had been any drift from the initial calibration (e.g., if scan lines from a tool feature were not perfectly aligned between representations of a tool in images captured from first and second image sensors). However, other approaches are also possible. For example, in some cases, known dimensions of tools depicted in images from the first and second image sensors may be used to convert a pixel offset value into spatial measurements, such as millimeters, and these spatial measurement values may be compared against a threshold for the surgical procedure to determine 204 the appropriateness of recalibration. For instance, in a case where the disclosed technology is used during a procedure which does not require sub-millimeter accuracy (e.g., hernia reconstruction), if the offset was a millimeter or less, then the determination 204 may indicate that recalibration is not appropriate, even though there had been some drift from the calibration values then being used. Other variations, such as using thresholds based on pixel offset values rather than spatial measurement offset values, are also possible, and will be immediately apparent to those of ordinary skill in the art in light of this disclosure. Accordingly, the above description of how it may be determined 204 whether recalibration is appropriate should be understood as being illustrative only, and should not be treated as limiting.

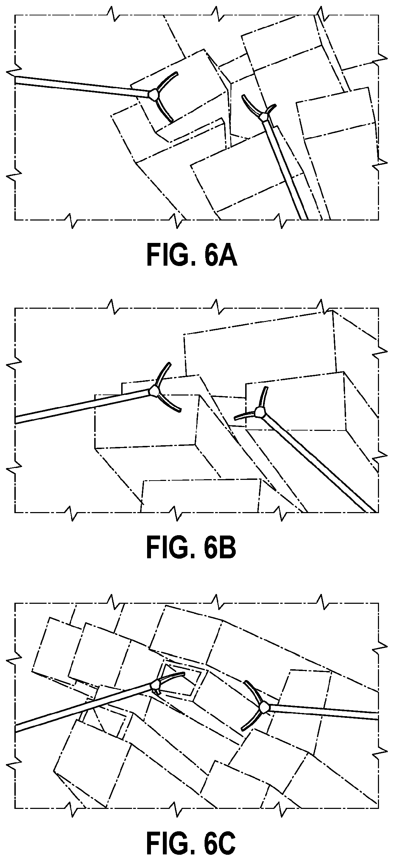

As shown in FIG. 2, if it was determined that there was no need for recalibration, then the previously derived 202 corrected images could be used to generate 205 3D information (e.g., a 3D reconstruction of the surgical site, triangulated locations in 3D of user specified points, tool tips or other points of interest), and the process could return to capture 201 further images. This could then be repeated throughout the surgical procedure. Alternatively, if it was determined 204 that recalibration was appropriate, then a method such as show in FIG. 2 could proceed with the performance 206 of at least one recalibration action. This may include, for example, requesting that a user of the system move one or more known tools in the field of view of a stereo camera, thereby allowing the system to capture images such as shown in FIGS. 6A, 6B and 6C in which the geometry of those tools could be used (instead of the checkerboard pattern) to recalibrate the camera in a manner such as described previously in the context of FIG. 1. Alternatively, in some cases, rather than requesting that the user move one or more known tools, a system performing a process such as shown in FIG. 2 may request that the user move the stereo camera to obtain images showing the relevant known tool from a variety of perspectives. As yet another variation, in some cases a system implemented based on this disclosure may be configured to store images from sensors of a stereo camera in a memory buffer as they are obtained 201, and could use images from the memory buffer in recalibrating the stereo camera, rather than requesting the user take some additional action to capture additional images. In any of the above cases though, after a stereo camera had been recalibrated, the calibration data used to derive 202 corrected images may be updated by being replaced with the new calibration data, and the process may continue through the use of that updated calibration data rather than the calibration data used to derive 202 the previous corrected images.

Other variations on potential implementations of the disclosed technology are also possible. For example, while FIGS. 4 and 5 depict evaluation of alignment based on vertical offsets, other offsets may be used, such as horizontal offsets in a case where image capture devices are vertically offset relative to each other. Similarly, while the example embodiments described herein are referenced as having two image sensors (e.g., left and right cameras making up a stereo image system), it should be understood that the system may have more than two image sensors. It should also be understood that, although the image sensors disclosed herein may be implemented as scope-based image sensors (e.g., image sensors used to capture images for a laparoscopic stereo camera), other types of image sensor may be used. Furthermore, calibration as described herein may take place outside as well as inside a patient.

As another possible type of variation, in some cases, rather than evaluating the need of/and or performing recalibrations based on characteristics of known tools, it may be possible perform those activities using other known objects in the surgical site. For example, in some embodiments, the system may obtain pre-operative information associated with the patient anatomy, such as, for example, pre-operative image data (e.g., magnetic resonance imaging (MRI), computed tomography (CT), Position emission tomography (PET/CT), ultrasound, radiography (x-ray), and the like). Thus, in some cases a system implemented based on this disclosure may use characteristics of known portions of patient anatomy, either instead of, or in addition to, known characteristics of known tools. Other variations are also possible, and will be immediately apparent to those of ordinary skill in light of this disclosure (e.g., performing online calibration using known objects in contexts other than surgical procedures). Alternatively, in some embodiments, functionality which was described above as being perform in the surgical site using characteristics of known tools may be performed externally. For example, in some cases if it was detected that recalibration was appropriate, a recalibration notice may be provided to a user requesting that he or she remove a stereo camera from the surgical site and take remedial actions, such as continuing the procedure with a different camera, or recalibrating the camera outside of the surgical site, such as through use of a convention calibration target such as shown in FIG. 1. Accordingly, the above description of how online calibration may be evaluated and/or performed in the context of a surgical procedure should be understood as being illustrative only, and should not be treated as limiting.

### III. Overview of Exemplary Surgical Visualization System

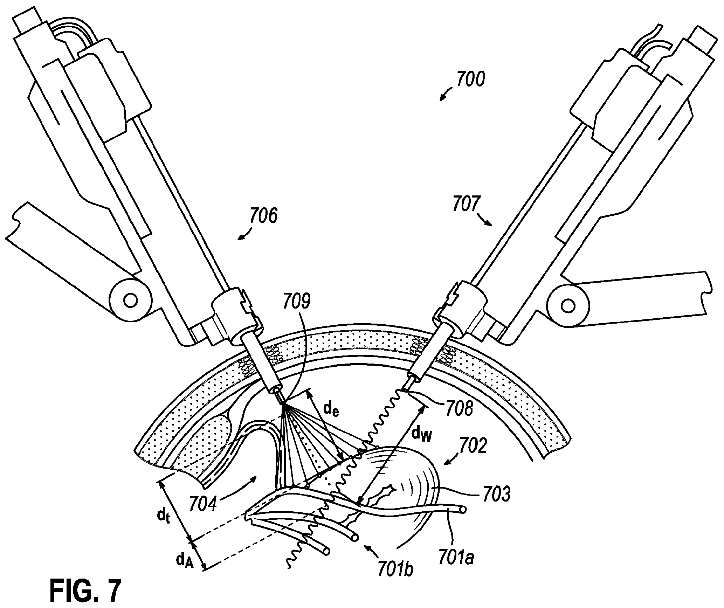

FIG. 7 depicts a schematic view of a surgical visualization system 700 according to at least one aspect of the present disclosure. The surgical visualization system 700 may create a visual representation of a critical structure 701a, 701b within an anatomical field. The surgical visualization system 700 may be used for clinical analysis and/or medical intervention, for example. In certain instances, the surgical visualization system 700 may be used intraoperatively to provide real-time, or near real-time, information to the clinician regarding proximity data, dimensions, and/or distances during a surgical procedure. The surgical visualization system 700 is configured for intraoperative identification of critical structure(s) and/or to facilitate the avoidance of critical structure(s) 701a, 701b by a surgical device. For example, by identifying critical structures 701a, 701b, a clinician may avoid maneuvering a surgical device into a critical structure 701a, 701b and/or a region in a predefined proximity of a critical structure 701a, 701b during a surgical procedure. The clinician may avoid dissection of and/or near a vein, artery, nerve, and/or vessel, for example, identified as a critical structure 701a, 701b, for example. In various instances, critical structure(s) 701a, 701b may be determined on a patient-by-patient and/or a procedure-by-procedure basis.

Critical structures 701a, 701b may be any anatomical structures of interest. For example, a critical structure 701a, 701b may be a ureter, an artery such as a superior mesenteric artery, a vein such as a portal vein, a nerve such as a phrenic nerve, and/or a sub-surface tumor or cyst, among other anatomical structures. In other instances, a critical structure 701a, 701b may be any foreign structure in the anatomical field, such as a surgical device, surgical fastener, clip, tack, bougie, band, and/or plate, for example. In one aspect, a critical structure 701a, 701b may be embedded in tissue. Stated differently, a critical structure 701a, 701b may be positioned below a surface of the tissue. In such instances, the tissue conceals the critical structure 701a, 701b from the clinician's view. A critical structure 701a, 701b may also be obscured from the view of an imaging device by the tissue. The tissue may be fat, connective tissue, adhesions, and/or organs, for example. In other instances, a critical structure 701a, 701b may be partially obscured from view. A surgical visualization system 700 is shown being utilized intraoperatively to identify and facilitate avoidance of certain critical structures, such as a ureter 701a and vessels 701b in an organ 702 (the uterus in this example), that are not visible on a surface 703 of the organ 702.

With continuing reference to FIG. 7, the surgical visualization system 700 incorporates tissue identification and geometric surface mapping, potentially in combination with a distance sensor system 704. In combination, these features of the surgical visualization system 700 may determine a position of a critical structure 701a, 701b within the anatomical field and/or the proximity of a surgical device 706 to the surface 703 of the visible tissue and/or to a critical structure 701a, 701b. The surgical device 706 may include an end effector having opposing jaws (not shown) and/or other structures extending from the distal end of the shaft of the surgical device 706. The surgical device 706 may be any suitable surgical device such as, for example, a dissector, a stapler, a grasper, a clip applier, a monopolar RF electrosurgical instrument, a bipolar RF electrosurgical instrument, and/or an ultrasonic instrument. As described herein, a surgical visualization system 700 may be configured to achieve identification of one or more critical structures 701a, 701b and/or the proximity of a surgical device 706 to critical structure(s) 701a, 701b.

The depicted surgical visualization system 700 includes an imaging system that includes an imaging device 707, such as a camera or a scope, for example, that is configured to provide real-time views of the surgical site. In various instances, an imaging device 707 includes a spectral camera (e.g., a hyperspectral camera, multispectral camera, a fluorescence detecting camera, or selective spectral camera), which is configured to detect reflected or emitted spectral waveforms and generate a spectral cube of images based on the molecular response to the different wavelengths. Views from the imaging device 707 may be provided to a clinician; and, in various aspects of the present disclosure, may be augmented with additional information based on the tissue identification, landscape mapping, and input from a distance sensor system 704. In such instances, a surgical visualization system 700 includes a plurality of subsystems-an imaging subsystem, a surface mapping subsystem, a tissue identification subsystem, and/or a distance determining subsystem. These subsystems may cooperate to intraoperatively provide advanced data synthesis and integrated information to the clinician(s).

The imaging device 707 of the present example includes an emitter 708, which is configured to emit spectral light in a plurality of wavelengths to obtain a spectral image of hidden structures, for example. The imaging device 707 may also include a three-dimensional camera and associated electronic processing circuits in various instances. In one aspect, the emitter 708 is an optical waveform emitter that is configured to emit electromagnetic radiation (e.g., near-infrared radiation (NIR) photons) that may penetrate the surface 703 of a tissue 702 and reach critical structure(s) 701a, 701b. The imaging device 707 and optical waveform emitter 708 thereon may be positionable by a robotic arm or a surgeon manually operating the imaging device. A corresponding waveform sensor (e.g., an image sensor, spectrometer, or vibrational sensor, etc.) on the imaging device 707 may be configured to detect the effect of the electromagnetic radiation received by the waveform sensor.

The wavelengths of the electromagnetic radiation emitted by the optical waveform emitter 708 may be configured to enable the identification of the type of anatomical and/or physical structure, such as critical structure(s) 701a, 701b. The identification of critical structure(s) 701a, 701b may be accomplished through spectral analysis, photo-acoustics, fluorescence detection, and/or ultrasound, for example. In one aspect, the wavelengths of the electromagnetic radiation may be variable. The waveform sensor and optical waveform emitter 708 may be inclusive of a multispectral imaging system and/or a selective spectral imaging system, for example. In other instances, the waveform sensor and optical waveform emitter 708 may be inclusive of a photoacoustic imaging system, for example. In other instances, an optical waveform emitter 708 may be positioned on a separate surgical device from the imaging device 707. By way of example only, the imaging device 707 may provide hyperspectral imaging in accordance with at least some of the teachings of U.S. Pat. No. 9,274,047, entitled "System and Method for Gross Anatomic Pathology Using Hyperspectral Imaging," issued March 1, 2016.

The depicted surgical visualization system 700 also includes an emitter 709, which is configured to emit a pattern of light, such as stripes, grid lines, and/or dots, to enable the determination of the topography or landscape of a surface 703. For example, projected light arrays may be used for three-dimensional scanning and registration on a surface 703. The projected light arrays may be emitted from an emitter 709 located on a surgical device 706 and/or an imaging device 707, for example. **In** one aspect, the projected light array is employed to determine the shape defined by the surface 703 of the tissue 702 and/or the motion of the surface 703 intraoperatively. An imaging device 707 is configured to detect the projected light arrays reflected from the surface 703 to determine the topography of the surface 703 and various distances with respect to the surface 703. By way of further example only, a visualization system 700 may utilize patterned light in accordance with at least some of the teachings of U.S. Pat. Pub. No. 2017/0055819, entitled "Set Comprising a Surgical Instrument," published March 2, 2017; and/or U.S. Pat. Pub No. 2017/0251900, entitled "Depiction System," published September 7, 2017.

The depicted surgical visualization system 700 also includes a distance sensor system 704 configured to determine one or more distances at the surgical site. In one aspect, the distance sensor system 704 may include a time-of-flight distance sensor system that includes an emitter, such as the structured light emitter 709; and a receiver (not shown), which may be positioned on the surgical device 706. In other instances, the time-of-flight emitter may be separate from the structured light emitter. In one general aspect, the emitter portion of the time-of-flight distance sensor system 704 may include a laser source and the receiver portion of the time-of-flight distance sensor system 704 may include a matching sensor. A time-of-flight distance sensor system 704 may detect the "time of flight," or how long the laser light emitted by the structured light emitter 709 has taken to bounce back to the sensor portion of the receiver. Use of a very narrow light source in a structured light emitter 709 may enable a distance sensor system 704 to determine the distance to the surface 703 of the tissue 702 directly in front of the distance sensor system 704.

Referring still to FIG. 7, a distance sensor system 704 may be employed to determine an emitter-to-tissue distance (dₑ) from a structured light emitter 709 to the surface 703 of the tissue 702. A device-to-tissue distance (dt) from the distal end of the surgical device 706 to the surface 703 of the tissue 702 may be obtainable from the known position of the emitter 709 on the shaft of the surgical device 706 relative to the distal end of the surgical device 706. In other words, when the distance between the emitter 709 and the distal end of the surgical device 706 is known, the device-to-tissue distance (dt) may be determined from the emitter-to-tissue distance (dₑ). In certain instances, the shaft of a surgical device 706 may include one or more articulation joints; and may be articulatable with respect to the emitter 709 and the jaws. The articulation configuration may include a multi-joint vertebrae-like structure, for example. In certain instances, a three-dimensional camera may be utilized to triangulate one or more distances to the surface 703.

As described above, a surgical visualization system 700 may be configured to determine the emitter-to-tissue distance (dₑ) from an emitter 709 on a surgical device 706 to the surface 703 of a uterus 702 via structured light. The surgical visualization system 700 is configured to extrapolate a device-to-tissue distance (dt) from the surgical device 706 to the surface 703 of the uterus 702 based on emitter-to-tissue distance (dₑ). The surgical visualization system 700 is also configured to determine a tissue-to-ureter distance (d_{A}) from a ureter 701a to the surface 703 and a camera-to-ureter distance (d_{w}), from the imaging device 707 to the ureter 701a. Surgical visualization system 700 may determine the camera-to-ureter distance (d_{w}), with spectral imaging and time-of-flight sensors, for example. In various instances, a surgical visualization system 700 may determine (e.g., triangulate) a tissue-to-ureter distance (d_{A}) (or depth) based on other distances and/or the surface mapping logic described herein.

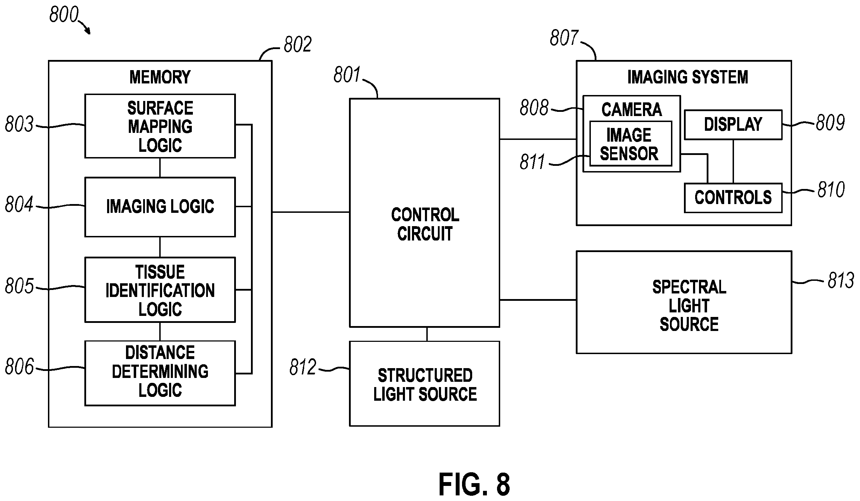

FIG. 8 is a schematic diagram of a control system 800, which may be utilized with a surgical visualization system 700. The depicted control system 800 includes a control circuit 801 in signal communication with a memory 802. The memory 802 stores instructions executable by the control circuit 801 to determine and/or recognize critical structures (e.g., critical structures 701a, 701b depicted in FIG. 7), determine and/or compute one or more distances and/or three-dimensional digital representations, and to communicate certain information to one or more clinicians. For example, a memory 802 stores surface mapping logic 803, imaging logic 804, tissue identification logic 805), or distance determining logic 806 or any combinations of logic 803, 804, 805, 806. The control system 800 also includes an imaging system 807 having one or more cameras 808 (like the imaging device 707 depicted in FIG. 7), one or more displays 809, one or more controls 810 or any combinations of these elements. The one or more cameras 808 may include one or more image sensors 811 to receive signals from various light sources emitting light at various visible and invisible spectra (e.g., visible light, spectral imagers, three-dimensional lens, among others). The display 809 may include one or more screens or monitors for depicting real, virtual, and/or virtually-augmented images and/or information to one or more clinicians.

In various aspects, a main component of a camera 808 includes an image sensor 811. An image sensor 811 may include a Charge-Coupled Device (CCD) sensor, a Complementary Metal Oxide Semiconductor (CMOS) sensor, a short-wave infrared (SWIR) sensor, a hybrid CCD/CMOS architecture (sCMOS) sensor, and/or any other suitable kind(s) of technology. An image sensor 811 may also include any suitable number of chips.

The depicted control system 800 also includes a spectral light source 812 and a structured light source 813. In certain instances, a single source may be pulsed to emit wavelengths of light in the spectral light source 812 range and wavelengths of light in the structured light source 813 range. Alternatively, a single light source may be pulsed to provide light in the invisible spectrum (e.g., infrared spectral light) and wavelengths of light on the visible spectrum. A spectral light source 812 may include a hyperspectral light source, a multispectral light source, a fluorescence excitation light source, and/or a selective spectral light source, for example. In various instances, tissue identification logic 805 may identify critical structure(s) via data from a spectral light source 812 received by the image sensor 811 portion of a camera 808. Surface mapping logic 803 may determine the surface contours of the visible tissue based on reflected structured light. With time-of-flight measurements, distance determining logic 806 may determine one or more distance(s) to the visible tissue and/or critical structure(s) 701a, 701b. One or more outputs from surface mapping logic 803, tissue identification logic 805, and distance determining logic 806, may be provided to imaging logic 804, and combined, blended, and/or overlaid to be conveyed to a clinician via the display 809 of the imaging system 807.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may be designed to be disposed of after a single use, or they may be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that may penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical visualization system comprising:
a) a stereo camera comprising a first image sensor and a second image sensor; and
b) a processor configured to obtain calibration data for the stereo camera and, during a surgical procedure, perform a set of three dimensional position determination tasks comprising:
i) capturing a first image of a surgical site from the first image sensor and a second image of the surgical site from the second image sensor;
ii) using the calibration data, deriving a first corrected image (410) based on the first image, and a second corrected image (420) based on the second image, wherein the first corrected image (410) and the second corrected image (420) each depict a surgical tool in the surgical site;
iii) determining an offset by performing one or more steps comprising identifying a feature in the depiction of the surgical tool in the first corrected image (410) and the depiction of the surgical tool in the second corrected image (420), and determining a distance between the feature in the depiction of the surgical tool in the first corrected image (410) and the feature in the depiction of the surgical tool in the second corrected image (420) in a direction perpendicular to a line segment connecting corresponding points in the first image sensor and the second image sensor;
iv) determining, based on the offset, whether the stereo camera requires recalibration;
v) in the event that the stereo camera is determined to require recalibration, performing at least one recalibration action; and
vi) determining, for each of one or more points in the surgical site, a three dimensional position of that point based on the calibration data and data from the first and second image sensors.

2. The system of claim 1, wherein:
a) the processor is configured perform a set of calibration image set creation tasks comprising capturing a first set of images depicting a known object in the surgical site using the first image sensor, and a second set of images depicting the known object in the surgical site using the second image sensor, wherein each image from the first set of images has a corresponding image from the second set of images captured simultaneously with that image from the first set of images; and
b) the at least one recalibration action comprises updating the calibration data based on the known object in the surgical site from the first set of images and the known object in the surgical site from the second set of images.

3. The system of claim 2, wherein:
a) the known object in the surgical site is a surgical tool;
b) the set of calibration image set creation tasks is comprised by the at least one recalibration action; and
c) the set of calibration image set creation tasks comprises presenting instructions to a user to move the surgical tool in the surgical site.

4. The system of claim 2, wherein:
a) the set of calibration image set creation tasks is comprised by the at least one recalibration action; and
b) the set of calibration image set creation tasks comprises presenting instructions to a user to obtain different views of the known object by moving the stereo camera.

5. The system of claim 2, wherein:
a) the surgical visualization system comprises a memory (802);
b) the processor is configured to repeatedly perform the set of three dimensional position determination tasks during the surgical procedure;
c) the processor is configured to capture the first set of images depicting the known object in the surgical site by, each time the task of capturing the first image of the surgical site from the first image sensor and the second image of the surgical site from the second image sensor is performed, storing the first image of the surgical from the first image sensor and the second image of the surgical site from the second image sensor in a buffer in the memory; and
d) the first set of images depicting the known object in the surgical site comprises images from the first image sensor in the buffer, and the second set of images depicting the known object in the surgical site comprises images from the second image sensor in the buffer.

6. The system of any preceding claim, wherein the at least one recalibration action comprises providing a recalibration notice to a user.

7. The system of claim 1, wherein:
a) the feature comprised by the surgical tool is a distal tip of the surgical tool; and
b) the line segment connecting corresponding points in the first image sensor connects a center of the first image sensor and a center of the second image sensor.

8. The system of claim 1, wherein determining, based on the offset, whether the stereo camera requires recalibration comprises:
a) converting the distance between the feature in the depiction of the surgical tool in the first corrected image and the feature in the depiction of the surgical tool in the second corrected image from pixels to millimeters based on a known dimension of the surgical tool; and
b) comparing the distance of in millimeters to a threshold associated with the surgical procedure.

9. The system of any preceding claim, wherein:
a) the at least one recalibration action comprises updating the calibration data; and
b) determining, for each of one or more points in the surgical site, the three dimensional position of that point based on the calibration data and data from the first and second image sensors comprises:
i) in the event that the stereo camera is determined not to require recalibration, determining, for each of one or more points in the surgical site, the three dimensional position of that point based on the first corrected image and the second corrected image; and
ii) in the event that the stereo camera is determined to require recalibration, determining, for each of one or more points in the surgical site, the three dimensional position of that point, based on a first new corrected image and a second new corrected image, wherein the first new corrected image and the second new corrected image are generated using the calibration data after updating the calibration data by performing the at least one recalibration action.

10. A processor-implemented method of recalibrating a stereo camera during a surgical procedure, the method comprising performing a set of three dimensional position determination tasks comprising:
a) capturing a first image of a surgical site from the first image sensor comprised by the stereo camera and a second image of the surgical site from a second image sensor comprised by the stereo camera;
b) using calibration data for the stereo camera, deriving a first corrected image based on the first image, and a second corrected image based on the second image, wherein the first corrected image and the second corrected image each depict a surgical tool in the surgical site;
c) determining an offset by performing one or more steps comprising identifying a feature in the depiction of the surgical tool in the first corrected image and the depiction of the surgical tool in the second corrected image, and determining a distance between the feature in the depiction of the surgical tool in the first corrected image and the feature in the depiction of the surgical tool in the second corrected image in a direction perpendicular to a line segment connecting corresponding points in the first image sensor and the second image sensor;
d) determining, based on the offset, whether the stereo camera requires recalibration; and
e) based on determining that the stereo camera requires recalibration, performing at least one recalibration action.

11. A non-transitory computer readable medium storing instructions operable to, when executed by a processor, cause a surgical visualization system to perform a set of three dimensional position determination tasks comprising:
a) capturing a first image of a surgical site from a first image sensor comprised by a stereo camera and a second image of the surgical site from a second image sensor comprised by the stereo camera;
b) using calibration data for the stereo camera, deriving a first corrected image based on the first image, and a second corrected image based on the second image, wherein the first corrected image (410) and the second corrected image (420) each depict a surgical tool in the surgical site;
c) determining an offset by performing one or more steps comprising identifying a feature in the depiction of the surgical tool in the first corrected image (410) and the depiction of the surgical tool in the second corrected image (420), and determining a distance between the feature in the depiction of the surgical tool in the first corrected image (410) and the feature in the depiction of the surgical tool in the second corrected image (420) in a direction perpendicular to a line segment connecting corresponding points in the first image sensor and the second image sensor;
d) determining, based on the offset, whether the stereo camera requires recalibration;
e) in the event that the stereo camera is determined to require recalibration, performing at least one recalibration action; and
f) determining, for each of one or more points in the surgical site, a three dimensional position of that point based on the calibration data for the stereo camera and data from the first and second image sensors.

12. The non-transitory computer readable medium of claim 11, wherein:
a) the instructions are operable to, when executed by the processor, cause the processor to perform a set of calibration image set creation tasks comprising capturing a first set of images depicting a known object in the surgical site using the first image sensor, and a second set of images depicting the known object in the surgical site using the second image sensor, wherein each image from the first set of images has a corresponding image from the second set of images captured simultaneously with that image from the first set of images; and
b) the at least one recalibration action comprises updating the calibration data for the stereo camera based on the known object in the surgical site from the first set of images and the known object in the surgical site from the second set of images.

13. The non-transitory computer readable medium of claim 11, wherein:
a) the first corrected image and the second corrected image each depict a surgical tool in the surgical site;
b) the surgical tool comprises a features; and
c) determining the offset by performing one or more steps comprising comparing the first corrected image and the second corrected image comprises:
i) identifying the feature in the depiction of the surgical tool in the first corrected image and the depiction of the surgical tool in the second corrected image; and
ii) determining a distance between the feature in the depiction of the surgical tool in the first corrected image and the feature in the depiction of the surgical tool in the second corrected image in a direction perpendicular to a line segment connecting corresponding points in the first image sensor and the second image sensor.

14. A computer program comprising instructions operable to, when executed by a processor, cause a surgical visualization system to perform a set of three dimensional position determination tasks comprising:
a) capturing a first image of a surgical site from a first image sensor comprised by a stereo camera and a second image of the surgical site from a second image sensor comprised by the stereo camera;
b) using calibration data for the stereo camera, deriving a first corrected image based on the first image, and a second corrected image based on the second image, wherein the first corrected image (410) and the second corrected image (420) each depict a surgical tool in the surgical site;
c) determining an offset by performing one or more steps comprising identifying a feature in the depiction of the surgical tool in the first corrected image (410) and the depiction of the surgical tool in the second corrected image (420), and determining a distance between the feature in the depiction of the surgical tool in the first corrected image (410) and the feature in the depiction of the surgical tool in the second corrected image (420) in a direction perpendicular to a line segment connecting corresponding points in the first image sensor and the second image sensor;
d) determining, based on the offset, whether the stereo camera requires recalibration;
e) in the event that the stereo camera is determined to require recalibration, performing at least one recalibration action; and
f) determining, for each of one or more points in the surgical site, a three dimensional position of that point based on the calibration data for the stereo camera and data from the first and second image sensors.

## Patentansprüche

1. Chirurgisches Visualisierungssystem, umfassend:
a) eine Stereokamera, die einen ersten Bildsensor und einen zweiten Bildsensor umfasst; und
b) einen Prozessor, der konfiguriert ist, um Kalibrierdaten für die Stereokamera zu erhalten und während eines chirurgischen Eingriffs einen Satz von Aufgaben zur dreidimensionalen Positionsbestimmung durchzuführen, umfassend:
i) Erfassen eines ersten Bildes eines Operationsorts von dem ersten Bildsensor und eines zweiten Bildes des Operationsorts von dem zweiten Bildsensor;
ii) unter Verwendung der Kalibrierdaten, Ableiten eines ersten korrigierten Bildes (410) basierend auf dem ersten Bild und eines zweiten korrigierten Bildes (420) basierend auf dem zweiten Bild, wobei das erste korrigierte Bild (410) und das zweite korrigierte Bild (420) jeweils ein chirurgisches Instrument im Operationsort darstellen;
iii) Bestimmen eines Versatzes durch Durchführen eines oder mehrerer Schritte, umfassend Identifizieren eines Merkmals in der Darstellung des chirurgischen Instruments in dem ersten korrigierten Bild (410) und der Darstellung des chirurgischen Instruments in dem zweiten korrigierten Bild (420), und Bestimmen eines Abstands zwischen dem Merkmal in der Darstellung des chirurgischen Instruments in dem ersten korrigierten Bild (410) und dem Merkmal in der Darstellung des chirurgischen Instruments in dem zweiten korrigierten Bild (420) in einer Richtung senkrecht zu einem Liniensegment, der entsprechende Punkte in dem ersten Bildsensor und dem zweiten Bildsensor verbindet;
iv) Bestimmen, basierend auf dem Versatz, ob die Stereokamera eine Neukalibrierung erfordert;
v) falls festgestellt wird, dass bestimmt wird, dass die Stereokamera eine Neukalibrierung erfordert, Durchführen mindestens einer Neukalibrierungsmaßnahme; und
vi) Bestimmen, für jeden von einem oder mehreren Punkten in dem Operationsort, einer dreidimensionalen Position dieses Punkts basierend auf den Kalibrierdaten und Daten von dem ersten und zweiten Bildsensor.

2. System nach Anspruch 1, wobei:
a) der Prozessor konfiguriert ist, um einen Satz von Erzeugungsaufgaben für Kalibrierbildsätze durchzuführen, umfassend das Erfassen eines ersten Satzes von Bildern, die ein bekanntes Objekt im Operationsort darstellen, unter Verwendung des ersten Bildsensors, und eines zweiten Satzes von Bildern, die das bekannte Objekt im Operationsort darstellen, unter Verwendung des zweiten Bildsensors, wobei jedes Bild aus dem ersten Satz von Bildern ein entsprechendes Bild aus dem zweiten Satz von Bildern aufweist, das gleichzeitig mit diesem Bild aus dem ersten Satz von Bildern erfasst wurde; und
b) die mindestens eine Neukalibrierungsmaßnahme das Aktualisieren der Kalibrierdaten basierend auf dem bekannten Objekt im Operationsort aus dem ersten Satz von Bildern und dem bekannten Objekt im Operationsort aus dem zweiten Satz von Bildern umfasst.

3. System nach Anspruch 2, wobei:
a) das bekannte Objekt in dem Operationsort ein chirurgisches Instrument ist;
b) der Satz von Erzeugungsaufgaben für Kalibrierbildsätze von der mindestens einen Neukalibrierungsmaßnahme umfasst wird; und
c) der Satz von Erzeugungsaufgaben für Kalibrierbildsätze das Bereitstellen von Anweisungen für einen Benutzer, das chirurgische Instrument in dem Operationsort zu bewegen, umfasst.

4. System nach Anspruch 2, wobei:
a) der Satz von Erzeugungsaufgaben für Kalibrierbildsätze von der mindestens einen Neukalibrierungsmaßnahme umfasst wird; und
b) der Satz von Erzeugungsaufgaben für Kalibrierbildsätze das Bereitstellen von Anweisungen für einen Benutzer, durch Bewegen der Stereokamera unterschiedliche Ansichten des bekannten Objekts zu erhalten, umfasst.

5. System nach Anspruch 2, wobei:
a) das chirurgische Visualisierungssystem einen Speicher (802) umfasst;
b) der Prozessor konfiguriert ist, um den Satz von Aufgaben zur dreidimensionalen Positionsbestimmung während des chirurgischen Eingriffs wiederholt auszuführen;
c) der Prozessor konfiguriert ist, um den ersten Satz von Bildern, die das bekannte Objekt in dem Operationsort darstellen, dadurch zu erfassen, dass jedes Mal, wenn die Aufgabe des Erfassens des ersten Bildes des Operationsorts von dem ersten Bildsensor und des zweiten Bildes des Operationsorts von dem zweiten Bildsensor ausgeführt wird, das erste Bild des Operationsorts von dem ersten Bildsensor und das zweite Bild des Operationsorts von dem zweiten Bildsensor in einem Puffer in dem Speicher gespeichert werden; und
d) der erste Satz von Bildern, die das bekannte Objekt in dem Operationsort darstellen, Bilder von dem ersten Bildsensor in dem Puffer umfasst, und der zweite Satz von Bildern, die das bekannte Objekt in dem Operationsort darstellen, Bilder von dem zweiten Bildsensor in dem Puffer umfasst.

6. System nach einem der vorstehenden Ansprüche, wobei die mindestens eine Neukalibrierungsmaßnahme das Bereitstellen eines Neukalibrierungshinweises an einen Benutzer umfasst.

7. System nach Anspruch 1, wobei:
a) das von dem chirurgischen Instrument umfasste Merkmal eine distale Spitze des chirurgischen Instruments ist; und
b) das Liniensegment, das entsprechende Punkte in dem ersten Bildsensor verbindet, ein Zentrum des ersten Bildsensors und ein Zentrum des zweiten Bildsensors verbindet.

8. System nach Anspruch 1, wobei das Bestimmen, basierend auf dem Versatz, ob die Stereokamera eine Neukalibrierung erfordert, Folgendes umfasst:
a) Umwandeln des Abstandes zwischen dem Merkmal in der Darstellung des chirurgischen Instruments in dem ersten korrigierten Bild und dem Merkmal in der Darstellung des chirurgischen Instruments in dem zweiten korrigierten Bild von Pixeln in Millimeter basierend auf einer bekannten Abmessung des chirurgischen Instruments; und
b) Vergleichen des Abstandes in Millimetern mit einem Schwellenwert, der dem chirurgischen Eingriff zugeordnet ist.

9. System nach einem der vorstehenden Ansprüche, wobei:
a) die mindestens eine Neukalibrierungsmaßnahme das Aktualisieren der Kalibrierdaten umfasst; und
b) Bestimmen, für jeden von einem oder mehreren Punkten an dem Operationsort, der dreidimensionalen Position dieses Punktes basierend auf den Kalibrierdaten und Daten von dem ersten und dem zweiten Bildsensor Folgendes umfasst:
i) falls festgestellt wird, dass die Stereokamera keine Neukalibrierung erfordert, Bestimmen, für jeden von einem oder mehreren Punkten an dem Operationsort, der dreidimensionalen Position dieses Punktes basierend auf dem ersten korrigierten Bild und dem zweiten korrigierten Bild; und
ii) falls festgestellt wird, dass die Stereokamera eine Neukalibrierung erfordert, Bestimmen, für jeden von einem oder mehreren Punkten an dem Operationsort, der dreidimensionalen Position dieses Punktes basierend auf einem ersten neuen korrigierten Bild und einem zweiten neuen korrigierten Bild, wobei das erste neue korrigierte Bild und das zweite neue korrigierte Bild unter Verwendung der Kalibrierdaten nach dem Aktualisieren der Kalibrierdaten durch Ausführen der mindestens einen Neukalibrierungsmaßnahme erzeugt werden.

10. Prozessorgestütztes Verfahren zum Neukalibrieren einer Stereokamera während eines chirurgischen Eingriffs, wobei das Verfahren das Ausführen eines Satzes von Aufgaben zur Bestimmung der dreidimensionalen Position umfasst, umfassend:
a) Erfassen eines ersten Bildes eines Operationsorts von dem ersten Bildsensor, der von der Stereokamera umfasst wird, und eines zweiten Bildes des Operationsorts von einem zweiten Bildsensor, der von der Stereokamera umfasst wird;
b) unter Verwendung von Kalibrierdaten für die Stereokamera, Ableiten eines ersten korrigierten Bildes basierend auf dem ersten Bild und eines zweiten korrigierten Bildes basierend auf dem zweiten Bild, wobei das erste korrigierte Bild und das zweite korrigierte Bild jeweils ein chirurgisches Instrument an dem Operationsort darstellen;
c) Bestimmen eines Versatzes durch Durchführen eines oder mehrerer Schritte, umfassend Identifizieren eines Merkmals in der Darstellung des chirurgischen Instruments in dem ersten korrigierten Bild und der Darstellung des chirurgischen Instruments in dem zweiten korrigierten Bild, und Bestimmen eines Abstands zwischen dem Merkmal in der Darstellung des chirurgischen Instruments in dem ersten korrigierten Bild und dem Merkmal in der Darstellung des chirurgischen Instruments in dem zweiten korrigierten Bild in einer Richtung senkrecht zu einem Liniensegment, der entsprechende Punkte in dem ersten Bildsensor und dem zweiten Bildsensor verbindet;
d) Bestimmen, basierend auf dem Versatz, ob die Stereokamera eine Neukalibrierung erfordert; und
e) basierend auf dem Bestimmen, dass die Stereokamera eine Rekalibrierung erfordert, Durchführen mindestens einer Neukalibrierungsmaßnahme.

11. Nichtflüchtiges computerlesbares Medium, das Anweisungen speichert, die bei Ausführung durch einen Prozessor ein chirurgisches Visualisierungssystem veranlassen, einen Satz von Aufgaben zur dreidimensionalen Positionsbestimmung auszuführen, umfassend:
a) Erfassen eines ersten Bildes eines Operationsorts von einem ersten Bildsensor, der von einer Stereokamera umfasst wird, und eines zweiten Bildes des Operationsorts von einem zweiten Bildsensor, der von der Stereokamera umfasst wird;
b) unter Verwendung von Kalibrierdaten für die Stereokamera, Ableiten eines ersten korrigierten Bildes basierend auf dem ersten Bild und eines zweiten korrigierten Bildes basierend auf dem zweiten Bild, wobei das erste korrigierte Bild (410) und das zweite korrigierte Bild (420) jeweils ein chirurgisches Instrument an dem Operationsort darstellen;
c) Bestimmen eines Versatzes durch Durchführen eines oder mehrerer Schritte, umfassend Identifizieren eines Merkmals in der Darstellung des chirurgischen Instruments in dem ersten korrigierten Bild (410) und der Darstellung des chirurgischen Instruments in dem zweiten korrigierten Bild (420), und Bestimmen eines Abstands zwischen dem Merkmal in der Darstellung des chirurgischen Instruments in dem ersten korrigierten Bild (410) und dem Merkmal in der Darstellung des chirurgischen Instruments in dem zweiten korrigierten Bild (420) in einer Richtung senkrecht zu einem Liniensegment, der entsprechende Punkte in dem ersten Bildsensor und dem zweiten Bildsensor verbindet;
d) Bestimmen, basierend auf dem Versatz, ob die Stereokamera eine Neukalibrierung erfordert;
e) falls festgestellt wird, dass bestimmt wird, dass die Stereokamera eine Neukalibrierung erfordert, Durchführen mindestens einer Neukalibrierungsmaßnahme; und
f) Bestimmen, für jeden von einem oder mehreren Punkten an dem Operationsort, einer dreidimensionalen Position dieses Punkts basierend auf den Kalibrierdaten für die Stereokamera und Daten von dem ersten und dem zweiten Bildsensor.

12. Nichtflüchtiges computerlesbares Medium nach Anspruch 11, wobei
a) die Anweisungen bei Ausführung durch den Prozessor den Prozessor veranlassen, einen Satz von Erzeugungsaufgaben für Kalibrierbildsätze durchzuführen, die das Erfassen eines ersten Satzes von Bildern, die ein bekanntes Objekt an dem Operationsort darstellen, unter Verwendung des ersten Bildsensors und eines zweiten Satzes von Bildern, die das bekannte Objekt an dem Operationsort darstellen, unter Verwendung des zweiten Bildsensors umfassen, wobei jedes Bild aus dem ersten Satz von Bildern ein entsprechendes Bild aus dem zweiten Satz von Bildern aufweist, das gleichzeitig mit diesem Bild aus dem ersten Satz von Bildern erfasst wird; und
b) die mindestens eine Neukalibrierungsmaßnahme das Aktualisieren der Kalibrierdaten für die Stereokamera basierend auf dem bekannten Objekt an dem Operationsort aus dem ersten Satz von Bildern und dem bekannten Objekt an dem Operationsort aus dem zweiten Satz von Bildern umfasst.

13. Nichtflüchtiges computerlesbares Medium nach Anspruch 11, wobei:
a) das erste korrigierte Bild und das zweite korrigierte Bild jeweils ein chirurgisches Instrument an dem Operationsort darstellen;
b) das chirurgische Instrument Merkmale umfasst; und
c) das Bestimmen des Versatzes durch Ausführen eines oder mehrerer Schritte, umfassend das Vergleichen des ersten korrigierten Bildes und des zweiten korrigierten Bildes, Folgendes umfasst:
i) Identifizieren des Merkmals in der Darstellung des chirurgischen Instruments in dem ersten korrigierten Bild und der Darstellung des chirurgischen Instruments in dem zweiten korrigierten Bild; und
ii) Bestimmen eines Abstands zwischen dem Merkmal in der Darstellung des chirurgischen Instruments in dem ersten korrigierten Bild und dem Merkmal in der Darstellung des chirurgischen Instruments in dem zweiten korrigierten Bild in einer Richtung senkrecht zu einer Streckenlinie, die entsprechende Punkte in dem ersten Bildsensor und dem zweiten Bildsensor verbindet.

14. Computerprogramm, umfassend Anweisungen, die bei Ausführung durch einen Prozessor ein chirurgisches Visualisierungssystem veranlassen, einen Satz von Aufgaben zur dreidimensionalen Positionsbestimmung auszuführen, umfassend:
a) Erfassen eines ersten Bildes eines Operationsorts von einem ersten Bildsensor, der von einer Stereokamera umfasst wird, und eines zweiten Bildes des Operationsorts von einem zweiten Bildsensor, der von der Stereokamera umfasst wird;
b) unter Verwendung von Kalibrierdaten für die Stereokamera, Ableiten eines ersten korrigierten Bildes basierend auf dem ersten Bild und eines zweiten korrigierten Bildes basierend auf dem zweiten Bild, wobei das erste korrigierte Bild (410) und das zweite korrigierte Bild (420) jeweils ein chirurgisches Instrument an dem Operationsort darstellen;
c) Bestimmen eines Versatzes durch Durchführen eines oder mehrerer Schritte, umfassend Identifizieren eines Merkmals in der Darstellung des chirurgischen Instruments in dem ersten korrigierten Bild (410) und der Darstellung des chirurgischen Instruments in dem zweiten korrigierten Bild (420), und Bestimmen eines Abstands zwischen dem Merkmal in der Darstellung des chirurgischen Instruments in dem ersten korrigierten Bild (410) und dem Merkmal in der Darstellung des chirurgischen Instruments in dem zweiten korrigierten Bild (420) in einer Richtung senkrecht zu einem Liniensegment, der entsprechende Punkte in dem ersten Bildsensor und dem zweiten Bildsensor verbindet;
d) Bestimmen, basierend auf dem Versatz, ob die Stereokamera eine Neukalibrierung erfordert;
e) falls festgestellt wird, dass bestimmt wird, dass die Stereokamera eine Neukalibrierung erfordert, Durchführen mindestens einer Neukalibrierungsmaßnahme; und
f) Bestimmen, für jeden von einem oder mehreren Punkten an dem Operationsort, einer dreidimensionalen Position dieses Punkts basierend auf den Kalibrierdaten für die Stereokamera und Daten von dem ersten und dem zweiten Bildsensor.

## Revendications

1. Système de visualisation chirurgicale comprenant :
a) une caméra stéréoscopique comprenant un premier capteur d'image et un second capteur d'image ; et
b) un processeur configuré pour obtenir des données d'étalonnage pour la caméra stéréoscopique et, pendant une intervention chirurgicale, effectuer un ensemble de tâches de détermination de position tridimensionnelle comprenant :
i) la capture d'une première image d'un site chirurgical à partir du premier capteur d'image et d'une seconde image du site chirurgical à partir du second capteur d'image ;
ii) à l'aide des données d'étalonnage, la dérivation d'une première image corrigée (410) sur la base de la première image, et d'une seconde image corrigée (420) sur la base de la seconde image, dans lequel la première image corrigée (410) et la seconde image corrigée (420) représentent chacune un outil chirurgical dans le site chirurgical ;
iii) la détermination d'un décalage en effectuant une ou plusieurs étapes comprenant l'identification d'une caractéristique dans la représentation de l'outil chirurgical dans la première image corrigée (410) et la représentation de l'outil chirurgical dans la seconde image corrigée (420), et la détermination d'une distance entre la caractéristique dans la représentation de l'outil chirurgical dans la première image corrigée (410) et la caractéristique dans la représentation de l'outil chirurgical dans la seconde image corrigée (420) dans une direction perpendiculaire à un segment de droite reliant des points correspondants dans le premier capteur d'image et le second capteur d'image ;
iv) le fait de déterminer, sur la base du décalage, si la caméra stéréoscopique nécessite un réétalonnage ;
v) dans le cas où il est déterminé que la caméra stéréoscopique nécessite un réétalonnage, réaliser au moins une action de réétalonnage ; et
vi) la détermination, pour chacun d'un ou de plusieurs points dans le site chirurgical, d'une position tridimensionnelle de ce point sur la base des données d'étalonnage et des données provenant des premier et second capteurs d'image.

2. Système selon la revendication 1, dans lequel :
a) le processeur est configuré pour effectuer un ensemble de tâches de création d'ensemble d'images d'étalonnage comprenant la capture d'un premier ensemble d'images représentant un objet connu dans le site chirurgical à l'aide du premier capteur d'image, et d'un second ensemble d'images représentant l'objet connu dans le site chirurgical à l'aide du second capteur d'image, dans lequel chaque image du premier ensemble d'images a une image correspondante provenant du second ensemble d'images capturée simultanément avec cette image provenant du premier ensemble d'images ; et
b) l'au moins une action de réétalonnage comprend la mise à jour des données d'étalonnage sur la base de l'objet connu dans le site chirurgical provenant du premier ensemble d'images et de l'objet connu dans le site chirurgical provenant du second ensemble d'images.

3. Système selon la revendication 2, dans lequel :
a) l'objet connu dans le site chirurgical est un outil chirurgical ;
b) l'ensemble de tâches de création d'ensembles d'images d'étalonnage est compris dans l'au moins une action de réétalonnage ; et
c) l'ensemble de tâches de création d'ensemble d'images d'étalonnage comprend la présentation d'instructions à un utilisateur pour déplacer l'outil chirurgical dans le site chirurgical.

4. Système selon la revendication 2, dans lequel :
a) l'ensemble de tâches de création d'ensemble d'images d'étalonnage est compris dans l'au moins une action de réétalonnage ; et
b) l'ensemble de tâches de création d'ensembles d'images d'étalonnage comprend la présentation d'instructions à un utilisateur pour obtenir différentes vues de l'objet connu en déplaçant la caméra stéréoscopique.

5. Système selon la revendication 2, dans lequel :
a) le système de visualisation chirurgicale comprend une mémoire (802) ;
b) le processeur est configuré pour effectuer de manière répétée l'ensemble de tâches de détermination de position tridimensionnelle pendant l'intervention chirurgicale ;
c) le processeur est configuré pour capturer le premier ensemble d'images représentant l'objet connu dans le site chirurgical en stockant, chaque fois que la tâche de capture de la première image du site chirurgical à partir du premier capteur d'image et de la seconde image du site chirurgical à partir du second capteur d'image est effectuée, la première image du chirurgical à partir du premier capteur d'image et la seconde image du site chirurgical à partir du second capteur d'image dans un tampon dans la mémoire ; et
d) le premier ensemble d'images représentant l'objet connu dans le site chirurgical comprend des images provenant du premier capteur d'image dans la mémoire tampon, et le second ensemble d'images représentant l'objet connu dans le site chirurgical comprend des images provenant du second capteur d'image dans la mémoire tampon.

6. Système selon l'une quelconque revendication précédente, dans lequel l'au moins une action de réétalonnage comprend la fourniture d'une notification de réétalonnage à un utilisateur.

7. Système selon la revendication 1, dans lequel :
a) la caractéristique comprise dans l'outil chirurgical est une pointe distale de l'outil chirurgical ; et
b) le segment de droite reliant des points correspondants dans le premier capteur d'image relie un centre du premier capteur d'image et un centre du second capteur d'image.

8. Système selon la revendication 1, dans lequel le fait de déterminer, sur la base du décalage, si la caméra stéréoscopique nécessite un réétalonnage comprend :
a) la conversion de la distance entre la caractéristique dans la représentation de l'outil chirurgical dans la première image corrigée et la caractéristique dans la représentation de l'outil chirurgical dans la seconde image corrigée de pixels en millimètres sur la base d'une dimension connue de l'outil chirurgical ; et
b) la comparaison de la distance en millimètres à un seuil associé à la procédure chirurgicale.

9. Système selon l'une quelconque revendication précédente, dans lequel :
a) l'au moins une action de réétalonnage comprend la mise à jour des données d'étalonnage ; et
b) la détermination, pour chacun d'un ou de plusieurs points dans le site chirurgical, de la position tridimensionnelle de ce point sur la base des données d'étalonnage et des données provenant des premier et second capteurs d'images comprend :
i) dans le cas où il est déterminé que la caméra stéréoscopique ne nécessite pas de réétalonnage, la détermination, pour chacun d'un ou de plusieurs points dans le site chirurgical, de la position tridimensionnelle de ce point sur la base de la première image corrigée et de la seconde image corrigée ; et
ii) dans le cas où il est déterminé que la caméra stéréoscopique nécessite un réétalonnage, la détermination, pour chacun d'un ou plusieurs points dans le site chirurgical, de la position tridimensionnelle de ce point, sur la base d'une première nouvelle image corrigée et d'une seconde nouvelle image corrigée, dans lequel la première nouvelle image corrigée et la seconde nouvelle image corrigée sont générées en utilisant les données d'étalonnage après la mise à jour des données d'étalonnage en effectuant l'au moins une action de réétalonnage.

10. Procédé mis en œuvre par processeur, permettant de réétalonner une caméra stéréoscopique pendant une intervention chirurgicale, le procédé comprenant l'exécution d'un ensemble de tâches de détermination de position tridimensionnelle comprenant :
a) la capture d'une première image d'un site chirurgical à partir du premier capteur d'image compris dans la caméra stéréoscopique et une seconde image du site chirurgical à partir d'un second capteur d'image compris dans la caméra stéréoscopique ;
b) l'utilisation de données d'étalonnage pour la caméra stéréoscopique, la dérivation d'une première image corrigée basée sur la première image, et d'une seconde image corrigée basée sur la seconde image, dans lequel la première image corrigée et la seconde image corrigée représentent chacune un outil chirurgical dans le site chirurgical ;
c) la détermination d'un décalage en effectuant une ou plusieurs étapes comprenant l'identification d'une caractéristique dans la représentation de l'outil chirurgical dans la première image corrigée et la représentation de l'outil chirurgical dans la seconde image corrigée, et la détermination d'une distance entre la caractéristique dans la représentation de l'outil chirurgical dans la première image corrigée et la caractéristique dans la représentation de l'outil chirurgical dans la seconde image corrigée dans une direction perpendiculaire à un segment de droite reliant des points correspondants dans le premier capteur d'image et le second capteur d'image ;
d) le fait de déterminer, en fonction du décalage, si la caméra stéréoscopique nécessite un réétalonnage ; et
e) sur la base de la détermination que la caméra stéréoscopique nécessite un réétalonnage, exécuter au moins une action de réétalonnage.

11. Support non transitoire lisible par ordinateur stockant des instructions qui, lorsqu'elles sont exécutées par un processeur, amènent un système de visualisation chirurgicale à effectuer un ensemble de tâches de détermination de position tridimensionnelle comprenant :
a) la capture d'une première image d'un site chirurgical à partir d'un premier capteur d'image compris dans une caméra stéréoscopique et d'une seconde image du site chirurgical à partir d'un second capteur d'image compris dans la caméra stéréoscopique ;
b) en utilisant des données d'étalonnage pour la caméra stéréoscopique, la dérivation d'une première image corrigée sur la base de la première image, et d'une seconde image corrigée sur la base de la seconde image, dans lequel la première image corrigée (410) et la seconde image corrigée (420) représentent chacune un outil chirurgical dans le site chirurgical ;
c) la détermination d'un décalage en effectuant une ou plusieurs étapes comprenant l'identification d'une caractéristique dans la représentation de l'outil chirurgical dans la première image corrigée (410) et la représentation de l'outil chirurgical dans la seconde image corrigée (420), et la détermination d'une distance entre la caractéristique dans la représentation de l'outil chirurgical dans la première image corrigée (410) et la caractéristique dans la représentation de l'outil chirurgical dans la seconde image corrigée (420) dans une direction perpendiculaire à un segment de droite reliant des points correspondants dans le premier capteur d'image et le second capteur d'image ;
d) le fait de déterminer, en fonction du décalage, si la caméra stéréoscopique nécessite un réétalonnage ;
e) dans le cas où il est déterminé que la caméra stéréoscopique nécessite un réétalonnage, l'exécution d'au moins une action de réétalonnage ; et
f) la détermination, pour chacun d'un ou de plusieurs points dans le site chirurgical, d'une position tridimensionnelle de ce point sur la base des données d'étalonnage pour la caméra stéréoscopique et des données provenant des premier et second capteurs d'image.

12. Support non transitoire lisible par ordinateur selon la revendication 11, dans lequel :
a) les instructions sont utilisables pour, lorsqu'elles sont exécutées par le processeur, amener le processeur à effectuer un ensemble de tâches de création d'ensembles d'images d'étalonnage comprenant la capture d'un premier ensemble d'images représentant un objet connu dans le site chirurgical à l'aide du premier capteur d'image, et d'un second ensemble d'images représentant l'objet connu dans le site chirurgical à l'aide du second capteur d'image, dans lequel chaque image du premier ensemble d'images a une image correspondante provenant du second ensemble d'images capturée simultanément avec cette image provenant du premier ensemble d'images ; et
b) l'au moins une action de réétalonnage comprend la mise à jour des données d'étalonnage pour la caméra stéréoscopique sur la base de l'objet connu dans le site chirurgical provenant du premier ensemble d'images et de l'objet connu dans le site chirurgical provenant du second ensemble d'images.

13. Support non transitoire lisible par ordinateur selon la revendication 11, dans lequel :
a) la première image corrigée et la seconde image corrigée représentent chacune un outil chirurgical dans le site chirurgical ;
b) l'outil chirurgical comprend une caractéristique ; et
c) la détermination du décalage en effectuant une ou plusieurs étapes comprenant la comparaison de la première image corrigée et de la seconde image corrigée comprend :
i) l'identification de la caractéristique dans la représentation de l'outil chirurgical dans la première image corrigée et la représentation de l'outil chirurgical dans la seconde image corrigée ; et
ii) la détermination d'une distance entre la caractéristique dans la représentation de l'outil chirurgical dans la première image corrigée et la caractéristique dans la représentation de l'outil chirurgical dans la seconde image corrigée dans une direction perpendiculaire à un segment de droite reliant des points correspondants dans le premier capteur d'image et le second capteur d'image.

14. Programme informatique comprenant des instructions qui, lorsqu'elles sont exécutées par un processeur, amènent un système de visualisation chirurgicale à effectuer un ensemble de tâches de détermination de position tridimensionnelle comprenant :
a) la capture d'une première image d'un site chirurgical à partir d'un premier capteur d'image compris dans une caméra stéréoscopique et d'une seconde image du site chirurgical à partir d'un second capteur d'image compris dans la caméra stéréoscopique ;
b) en utilisant des données d'étalonnage pour la caméra stéréoscopique, la dérivation d'une première image corrigée sur la base de la première image, et d'une seconde image corrigée sur la base de la seconde image, dans lequel la première image corrigée (410) et la seconde image corrigée (420) représentent chacune un outil chirurgical dans le site chirurgical ;
c) la détermination d'un décalage en effectuant une ou plusieurs étapes comprenant l'identification d'une caractéristique dans la représentation de l'outil chirurgical dans la première image corrigée (410) et la représentation de l'outil chirurgical dans la seconde image corrigée (420), et la détermination d'une distance entre la caractéristique dans la représentation de l'outil chirurgical dans la première image corrigée (410) et la caractéristique dans la représentation de l'outil chirurgical dans la seconde image corrigée (420) dans une direction perpendiculaire à un segment de droite reliant des points correspondants dans le premier capteur d'image et le second capteur d'image ;
d) le fait de déterminer, en fonction du décalage, si la caméra stéréoscopique nécessite un réétalonnage ;
e) dans le cas où il est déterminé que la caméra stéréoscopique nécessite un réétalonnage, l'exécution d'au moins une action de réétalonnage ; et
f) la détermination, pour chacun d'un ou de plusieurs points dans le site chirurgical, d'une position tridimensionnelle de ce point sur la base des données d'étalonnage pour la caméra stéréoscopique et des données provenant des premier et second capteurs d'image.
